# EUROPEAN PATENT APPLICATION

(11) **EP 4 781 937 A1**
(43) Date of publication of application: **29.07.2026**
(21) Application number: 25154243.7
(22) Date of filing: 27.01.2025
(51) Int. Cl.: A61B 18/18, A61B 18/20, A61B 18/00

(54) **SKIN TREATMENT DEVICE**

(71) Applicant: Braun GmbH, 61476 Kronberg im Taunus (DE)
(72) Inventor: GRIESHABER, Frieder, 61476 Kronberg (DE)
(74) Representative: P&G Patent Germany

(57) **Abstract**

The present disclosure is concerned with a skin treatment device having a treatment light source, a light emission window comprising a window element arranged for getting into skin contact during operation, a first sensor arranged for providing a first sensor signal indicative of an amount of hemoglobin in a skin area underneath the light emission window, and a processor coupled with the first sensor for communicating the first sensor signal in a user-perceptible manner by means of an indicator and/or for controlling a function of the skin treatment device in dependence on the first sensor signal.

## Description

### FIELD OF THE INVENTION

The present application is concerned with a skin treatment device that has a treatment light source, a light emission window and a sensor.

### BACKGROUND OF THE INVENTION

It is known that skin can be treated with light, specifically with light in the visible spectrum and in the near infrared spectrum. Low intensity light irradiation may be used to treat skin issues like acne. Light irradiation may last from seconds to many minutes or even hours. High intensity light irradiation may be used to manage hair growth. Light irradiation may last from microseconds over milliseconds to up to one second. Specifically, light applied onto the skin in a short time such as 0.1 ms to 300 ms at a fluence in between 0.5 J/cm² to 10 J/cm² becomes absorbed by the various skin components, thus heating the absorbers/chromophores and the skin in general to a level suitable for at least triggering premature transition of the hair follicle to the catagen stage (which will be followed by telogen stage in which the hair does not grow). Absorption of light by melanin may even lead to coagulation of proteins, even though this may not necessarily be intended by home-use devices. To achieve hair growth management, light must be absorbed by the melanin in the hair root to cause the mentioned premature catagen phase. To achieve the needed temperature for protein denaturation, a certain light amount needs to be absorbed in a certain period. The needed fluence is influenced by skin color and the melanin content in the hair, i.e., hair color. Other skin material such as hemoglobin, water etc. may absorb and/or scatter the light.

It is an object to provide a skin treatment device and a method of cosmetic skin treatment where the needed fluence can be reduced.

### SUMMARY OF THE INVENTION

In accordance with at least one aspect, a skin treatment device is provided that comprises a treatment light source, a light emission window comprising a window element arranged for getting into skin contact during operation, a first sensor arranged for providing a first sensor signal indicative of an amount of hemoglobin in a skin area underneath the light emission window, and a processor coupled with the first sensor for communicating the first sensor signal in a user-perceptible manner by means of an indicator and/or for controlling a function of the skin treatment device in dependence on the first sensor signal.

In accordance with at least one aspect, a method of applying light to skin for achieving a cosmetic effect, the method comprising the steps of (a pushing a light emission window of a skin treatment device, the light emission window comprising a window element, against the skin, (b) measuring a first sensor signal indicative of an amount of hemoglobin present in at least a portion of the skin underneath the light emission window, and (c1) indicating the determined sensor signal in a user-perceptible manner, and/or (c2) controlling a function of the skin treatment device in dependence on the determined sensor signal.

### BRIEF DESCRIPTION OF THE DRAWINGS

The present disclosure will be further elucidated by a detailed description of example embodiments and with reference to figures. In the figures
- Fig. 1: is a graph showing the absorption coefficient α for melanin, hemoglobin and oxyhemoglobin plotted against the wavelength λ of light;
- Fig. 2: is a schematic depiction of an example skin treatment device realized as an IPL (intense pulsed light) device suitable for hair growth management;
- Fig. 3: is a schematic depiction of a cut-out portion of the human skin from epidermis to subcutis including a hair growing in the skin;
- Fig. 4: is a graph showing the normalized intensity I_{N} of the light emitted by a xenon flash lamp as may be used in a skin treatment device;
- Fig. 5: is a schematic depiction of an example skin treatment device in accordance with the present disclosure; and
- Fig. 6: is a schematic depiction of an example head portion of a skin treatment device in accordance with the present disclosure.

### DETAILED DESCRIPTION OF THE INVENTION

A skin treatment device as proposed herein typically comprises a treatment light source such as one or several LEDs, one or several laser diodes, and/or one or several flash lamps. The skin treatment device may have a light emission window comprising a window element arranged for getting into skin contact during operation. The window element might be transmissive for the treatment light or even transparent. Further, the skin treatment device may comprise a first sensor arranged for providing a first sensor signal indicative of an amount of hemoglobin in a skin area underneath the light emission window. As will be discussed in detail below, it may be sensible to operate the device in such a way that for various situations the blood comprising hemoglobin is squeezed out from the irradiated skin area, specifically where different pressure is applied depending on the circumstances. A first sensor for determining the current hemoglobin content may thus be present. Finally, a processor coupled with the first sensor may be used for communicating a first sensor signal in a user-perceptible manner by means of an indicator. Additionally or alternatively, the processor may be arranged for controlling a function of the skin treatment device in dependence on the first sensor signal. An indicator may be helpful for a user operating the device so that the user is continuously guided during the operation to use the device in an improved or at least recommended manner. The processor may also control the skin treatment device in a predetermined manner in dependence on the first sensor signal, i.e., in dependence on the hemoglobin content. Examples of how the skin treatment device may be controlled by the processor include modifying one or more parameters such as fluence and/or pulse length, inhibiting the emission of treatment light from the treatment light source, and/or switching on or off one or more light filters such as one or more cut-off filters and/or one or more band-pass filters. Other controls may be mentioned in the below or may become evident to the skilled reader from the present disclosure.

In accordance with some aspects, the above-mentioned window element has a convex outer shape that can be pushed onto the skin to be treated. The convex curvature may be present in one direction but also in two, e.g., the outer shape may as one example follow a portion of a cylinder or as another example a portion of a sphere. But "convex" shall not be understood to be limited to circular/cylindrical and/or spherical shapes. The treatment-light transmissive window element may be made from glass, e.g., sapphire glass, or alternatively from a suitable plastic material. This shall not exclude other materials that are at least partially transparent and/or translucent for the treatment light.

In accordance with some aspects, a housing of the skin treatment device around the window element may follow the curvature of the window element, in particular such that there is an essentially smooth transition between the window element and the housing. This shall not exclude that there is a step or curvature difference between the window element and the housing.

In accordance with some aspects, the above-mentioned first sensor may comprise an optical sensor that has at least a first light source (also named: first sensor light source) and at least a first light-sensitive detector. The first sensor light source that may be monochromatic or essentially monochromatic. The first sensor light source may in operation emit first sensor light that is backscattered by the skin towards the first light-sensitive detector. The amount of light measured by the first light-sensitive detector may be indicative of the amount of hemoglobin present underneath the window element. The first sensor light source may be a monochromatic light source such as an LED or a laser diode. Alternatively, the first sensor light source may be a broad-band light source such as a flash lamp that comprises a band-pass filter. Independent from the kind of light source, the first sensor light source may emit light either limited to the wavelength range of between 380 nm and 620 nm such as 580 nm or limited to the wavelength range of between 620 nm and 1200 nm such as 680 nm. Additionally, the first sensor may comprise a second light source (also named: second sensor light source) that may be a monochromatic or essentially monochromatic light source such as an LED or laser diode. Alternatively, the second sensor light source may be a broad-band light source such as a flash lamp that comprises a band-pass filter. The second sensor light source may emit light limited to the wavelength range of between 380 nm and 620 nm such as 580 nm or limited to the range of between 620 nm and 1200 nm such as 680 nm. Additionally or alternatively, the first sensor may comprise at least one force sensor for measuring the force with which the window element is pushed against a treatment surface. Instead of measuring the hemoglobin content directly by an optical sensor, the hemoglobin content and the hemoglobin flow reduction in the different plexuses (see further below) may be indirectly derived from the applied pressure.

In accordance with some aspects, the skin treatment device may comprise at least one of the following additional features:
- A second sensor for measuring the skin color (aka: skin tone) and/or for measuring the hair color.
- A user interface allowing a user to input data into the user interface, the data may comprise at least one of the skin color / skin tone or hair color.

In accordance with some aspects, the above-mentioned processor may be arranged to determine an amount of hemoglobin present in the skin underneath the light emission window comprising the window element based on at least the first sensor signal (the first sensor signal may comprise data from several sensors). The processor may also be arranged to determine an achieved hemoglobin flow reduction, e.g., based on the first sensor signal comprising pressure data, and/or a blood flow reduction in at least two different plexuses of the skin underneath the light emission window. The processor may further be arranged to compare the determined hemoglobin content or amount and/or determined blood flow reduction with one or several respective threshold values / amounts. The one or several threshold values / amounts may be predefined and/or may depend on a measured or determined or inputted skin color / skin tone and/or hair color information. The processor may then be arranged to inhibit an emission of treatment light from the treatment light source, e.g., when the determined amount of hemoglobin present in the skin underneath the light emission window is above the threshold amount of hemoglobin or when the achieved blood flow reduction is below the threshold value.

Additionally or alternatively, the processor may be arranged to determine a force value with which the window element is pushed against the skin and in particular to compare the determined force value with a threshold force value with which the window element shall be pushed against the skin. The threshold value for the force may depend on at least on one of skin color / skin tone or hair color and may be predefined.

The processor may be arranged to indicate in a user-perceptible manner, e.g., by at least one or several audible, visual and/or tactile signals, at least one of a force with which the window element is currently pushed against the skin, the force value with which the window element shall be pushed against the skin and a sufficiency of a force currently being applied for achieving the intended amount of hemoglobin in the treated skin area and/or for achieving the intended level of blood flow reduction. The processor may be arranged to inhibit an emission of treatment light in case that the force value with which the window element is pushed against the skin is below the threshold force value.

The window element may be cooled, e.g., by a Peltier cooling element (TEC) and/or by air cooling. A cooling element may be provided that is in contact with the window element.

A skin treatment device as described herein may be used by pushing the device against the treatment surface such as human skin to be treated and by modifying a pressure applied by the user onto skin in accordance with a signal from an indicator offered in a user perceptible manner. The indicated user perceptible signal thus continuously guides the user in the ongoing recommended operation of the skin treatment device to change the way of operation towards a potentially improved skin treatment outcome. The use of the device may include a manual initiation of the treatment light emission, even though the device may be arranged to emit treatment light, e.g., in the form of a treatment light pulse, in an automated manner.

A method of applying light to skin for achieving a cosmetic effect may comprise the steps:
- Pushing a light emission window comprising a window element, specifically a convexly shaped window element, against the treatment surface, typically against the skin of a human being. Using a first sensor for determining a first sensor signal.
- Providing the first sensor signal indicative of an amount of hemoglobin in the skin being treated, i.e., in the skin underneath the light transmission window comprising the window element, and/or of the blood flow reduction achieved in the skin, specifically in at least two different layers of the skin for further processing.
- Indicating the first sensor signal in a user perceptible manner, e.g., in a visual, audible or tactile manner, and/or controlling a function of the skin treatment device in dependence on the determined first sensor signal (see functions mentioned above).

In the context of the present application, the term "hemoglobin" shall mean "hemoglobin and/or oxyhemoglobin" where this is not differently indicated or transparent from the description.

In a basic approach, human skin can be segmented into the epidermis (upper skin layer), the dermis (center skin layer) and the subcutis (deep skin layer). The vasculature supplying the skin with blood is divided into three distinct portions (plexuses): deep dermal plexus or deep arteriovenal plexus or subcutaneous plexus, cutaneous plexus or reticular plexus, and papillary plexus or superficial arteriovenal plexus or upper plexus. The upper plexus may be said to be located directly under the epidermis. The deep plexus supplies blood to the hair papilla. The cutaneous plexus may be said to be distributed around hairs without necessarily being responsible for supplying blood to the hair itself. The epidermis is the top layer of the skin and does essentially not comprise any hemoglobin. The epidermis comprises melanocytes that produce melanin (eumelanin and pheomelanin) that is responsible for the skin color. Additionally, the hair follicle comprises melanocytes that bind melanin into the hair, specifically in the hair root.

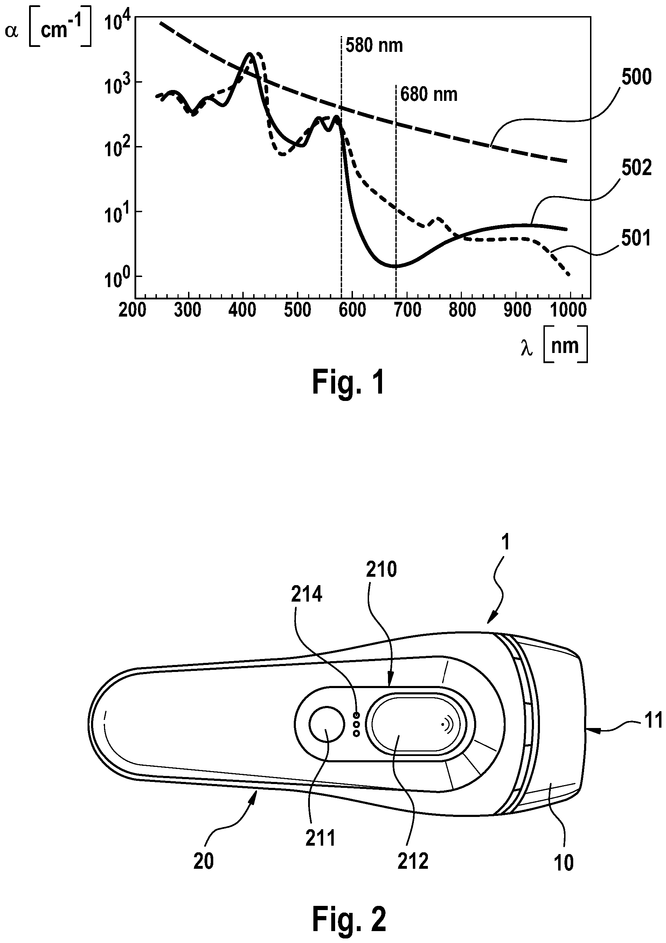

Melanin and hemoglobin are the two most important chromophores in the skin that absorb light. Typical absorption coefficients in dependence on the wavelength are shown in Fig. 1. The absolute absorption depends on the mass of the absorbing chromophores. Typically, much more hemoglobin is present in the skin than melanin. While the melanin content in the skin and the hair depends on the individual human, blood content may vary but has a less individual amount. In contrast to melanin, the blood content in the irradiated skin area can be influenced, e.g., by applying pressure to squeeze out blood from the irradiated area. This will be discussed in more detail in the following. Also, other components in the skin absorb but may also scatter light, e.g., collagen fibers may mainly scatter light in the visual and NIR wavelength range to 1000 nm. Thus, the amount of light present in a certain depth of the skin, e.g., at the level of the hair bulb, depends on the amount already absorbed in the upper layers of the skin and the amount scattered away.

To manage hair growth in the skin, it is necessary to heat the cells of the hair matrix so that the hair follicle is prematurely put into catagen phase, eventually leading to the hair falling out. This shall not rule out that proteins responsible for hair growth may eventually also become denaturized / coagulated. Light needs to be delivered to the dermal level where the hair matrix is located in the hair bulb. On the way to the respective dermal level, light may become absorbed by the melanin in the epidermis and by the hemoglobin in the papillary plexus and the cutaneous plexus as already mentioned. To provide the right light intensity at the hair matrix / hair root, the fluence of the light pulse applied by an IPL device needs to be increased the more light is absorbed by melanin and/or blood prior to reaching the lower lying hair matrix. This becomes not possible if the melanin content in the epidermis is too high as then the melanin particles in the skin heat too strongly and the risk of damage to the skin occurs.

In contrast to the melanin content in the skin, the hemoglobin content can at least partially be controlled, e.g., by applying pressure onto the skin as was already mentioned. Depending on the amount of pressure, blood can be removed from different levels of the skin. A low pressure can squeeze out the blood from the upper plexus (also called the superficial arteriovenal / vascular plexus). Increased pressure can additionally squeeze out the blood from the reticular or cutaneous plexus. This shall here mean the network of vasculature in which blood flows around the hair root. Increasing the pressure even further may squeeze out the blood from the deeper plexus supplying the hair root (also called the deep arteriovenous / vascular plexus). For the latter the intended effect is to stop the blood flow into the hair papilla. Where here "hair root" is mentioned, this applies essentially to the hair bulb, where the hair matrix cells are located, typically in close spatial relationship to the hair papilla. Removing blood, i.e., hemoglobin from the upper plexus and potentially also from the reticular plexus leads to a certain control of the amount of light that reaches the hair matrix and that heats the blood and thus the tissue. Specifically, removing hemoglobin from the upper plexus reduces the heating of the upper skin portion and more importantly reduces the absorption of light in the upper dermis so that a higher percentage of light reaches deeper skin layers and thus eventually the hair matrix.

In fair skin (e.g., Fitzpatrick skin typing (FST) I or II - pale to lightly colored) the reduction of hemoglobin as an absorber means that a higher percentage of light irradiated onto the skin reaches the hair root. Light may reach the hair bulb without prior interaction with the tissue or by becoming scattered, where scattered light may eventually also reach the hair bulb if it is not absorbed or finally scattered away. This reduction of hemoglobin as an absorber is specifically providing a benefit if the hair color is dark, e.g., brownish such as medium to dark brown. The melanin in the hair root is then the main absorbing chromophore in the skin. That means, e.g., that the fluence of the light to be irradiated onto the skin can be reduced as the heat generation due to absorbed light necessary for protein denaturation is mainly occurring in the hair root / hair bulb. Further, also for other skin types (Fitzpatrick scale FST III, IV, V and VI) the reduction of blood as an absorber is helpful to reduce the needed fluence for such dark hairs (i.e., brownish hair color). But, specifically for Fitzpatrick scale FST V and IV it may then be helpful to not squeeze out the blood from the capillaries supplying the hair root. The blood flowing through the hair papilla, which is surrounded by the hair bulb comprising the hair matrix cells, absorbs light and contributes to the heating of the hair bulb / hair matrix.

In fair skin (FST I and II) comprising hairs having a lighter color, e.g., lighter brownish such as dark blond or even blond, it may be sensible to not apply too much pressure and to not squeeze out the blood from the reticular plexus that causes the follicular perfusion. That implies that still pressure may be applied to squeeze out the blood from the upper plexus. The reticular plexus comprises capillaries and blood vessels that surround the hair root / hair bulb and thus heat the tissue surrounding the hair bulb / hair matrix. Heating this tissue reduces the temperature gradient between hair bulb and tissue and thus decreases the velocity of the heat transfer from the hair bulb into the neighboring tissue. The same applies also to tanned skin (e.g. FST III and IV) comprising such lighter colored hairs. In the latter case, additional measures of selecting the light wavelength and/or applying a cut-off filter and/or applying light with increased pulse length may be sensible.

The above discussed application of pressure to reduce blood flow, i.e., to squeeze out blood from the irradiated skin volume, is indicated in Table 1.

| Hair colour | | Dark | | Dark blond | Blond | Blond |
|---|---|---|---|---|---|---|
| Skin tone | | Fair skin FST I - II | Darker skin (FST > V) | Fair skin FST I - II | Fair skin FST I - II | Tanned skin FST III - IV |
| Reduced blood flow | Superficial arteriovenous plexus | ↓ | ↓ | ↓ | ↓ | ↓ |
| | Follicular perfusion / reticular plexus | ↓ | ↓ | ↓ | - | - |
| | Deep arteriovenous plexus | ↓ | - | - | - | - |
| Overall pressure | | ↑↑↑ | ↑↑ | ↑ | ↑ | ↑ |

Table 1: This table summarizes the pressure application for various situations. Hair color is indicated in the top row, skin tone in the second row. In the following three rows generally indicating "reduced blood flow", the reduction is indicated by a downwards pointing arrow, when the respective blood is to be reduced by applying pressure. This results in the overall pressure to be applied as indicated in the last row by upwards indicating arrows, where the pressure is to be higher the higher the number of arrows is.

The light source that may be used in a skin treatment device can be manifold and may range from regular lamps, LEDs, laser diodes to controlled flash lamps such as Xenon flash lamps. The light source being used shall be suitable for applying the needed fluence in the given time / pulse length and the needed wavelength or wavelength range. With light sources irradiating a broad spectrum such as flash lamps, one or several light filters may be used.

Fig. 1 is a depiction of three light absorption coefficient curves 500, 501 and 502 plotted against the wavelength. The wavelength λ is given in nanometer (nm) and the absorption coefficient α is given in 1/cm. The curves are plotted for a unit amount of material, i.e., the light absorption of the respective materials must be weighted by their amount. In the shown graph, curve 500 is the light absorption coefficient of melanin, curve 501 is the light absorption coefficient for hemoglobin and curve 502 is the light absorption coefficient for oxyhemoglobin. These are the most relevant absorbing materials in human skin for the discussion in the present application. Other materials in the human skin absorb light as well such as water and lipid etc. The curves may be misleading as there may be a higher amount of hemoglobin in the skin then melanin, but the depth location of the absorber also plays a crucial role as light that was already absorbed is not available anymore and typically melanin can be found in the epidermis, i.e., the upper level of the skin.

For the discussion in the present disclosure, it is also important to know that light penetrating through skin may become scattered instead of being absorbed. That means that light that shall reach, e.g., a hair root to become absorbed, may be scattered away. Light may also be backscattered and may then eventually reach the hair root. For light that is scattered and backscattered, the reduction of hemoglobin around the hair root may be relevant if such light shall reach the hair root prior to becoming absorbed.

Fig. 2 is a schematic depiction of a skin treatment device 1 that may be suitable for achieving hair growth management. The skin treatment device 1 comprises a head portion 10 that has a light emission window 11 for emitting light onto skin. While not shown, the light emission window may comprise a flat window element. As is usual in the art, the head portion 10 may comprise one or several sensors that allow controlling light emission. E.g., the head portion 10 may comprise skin contact sensors and light emission may only be allowed in case the complete light emission window 11 is in contact with the skin. This may be particularly relevant for high intensity light emission, where the emitted light pulse may be harmful for the human eye if the light pulse were emitted directly into the eye instead to the skin. Other sensors are also known in the art such as one or several skin tone sensors. A skin tone sensor may be used to adapt the light fluence and potentially other parameters in dependence to the determined skin tone value. Skin tone may be typically characterized by the Fitzpatrick scale, where a Fitzpatrick scale I relates to the palest skin and Fitzpatrick scale VI relates to deeply pigmented brown to darkest brown skin. Home use skin treatment devices for effecting hair growth management, typically realized as so-called IPL devices, where IPL stands for Intense Pulsed Light, are usually not suitable to treat skin having a Fitzpatrick scale VI as the high melanin content in the epidermis will heat the skin too strongly.

The skin treatment device 1 comprises a handle section 20 that here comprises an interface area 210 that comprises a first user-operable button 211 for setting one or several parameters of the device and a second user-operable button 212 for effecting the emission of a treatment light pulse (specifically in case the relevant conditions are fulfilled such as complete skin contact and skin having a Fitzpatrick scale V or below). Additionally, one or several indicators 214 are here present in the interface area 210. The shown example embodiment is not to be understood as limiting. The interface area may be spread over the outside surface of the skin treatment device and one or a plurality, e.g. three or more, of user-operable input elements may be provided. Further, one or a plurality of indicators may be used. An indicator may be arranged to provide user-perceptible information in a visual, audible and/or haptic/tactile manner. An indicator may comprise a display for providing visual information.

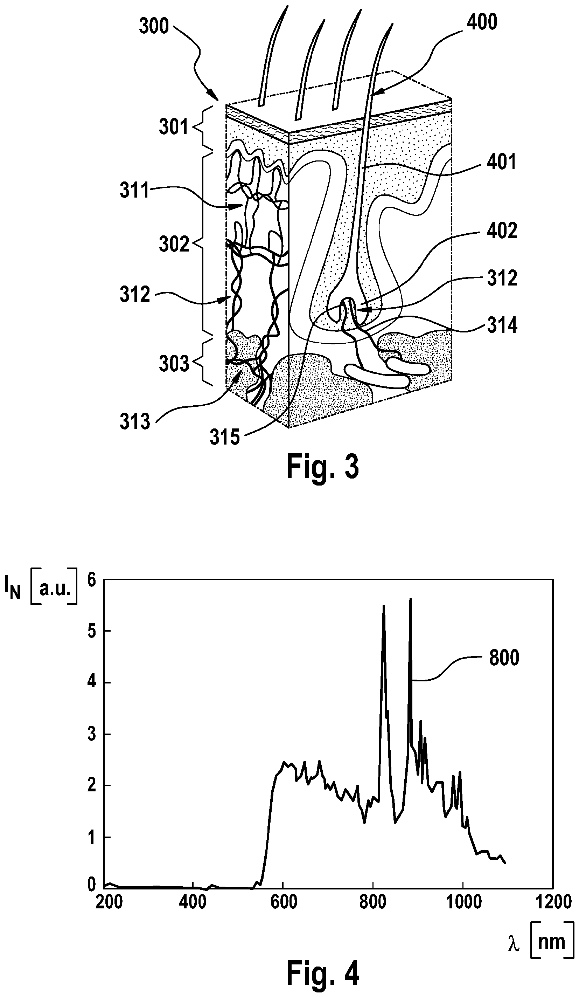

Fig. 3 is a schematic cross-sectional cut through human skin 300. A hair 400 is shown that grows in the skin 300. The hair 400 comprises a hair root 401 that is located within the skin 300 and a hair bulb 402 that represents the deepest portion of the hair root 401. The hair matrix, i.e., the part of the hair root 401 relevant for hair growth, is part of the hair bulb 402. In order to manage hair growth, it is relevant to control the heating of the hair matrix proteins in the hair bulb. The hair bulb 402 envelopes a hair papilla 315. An uppermost skin layer 301 is the epidermis, followed by a middle skin layer 302 being the dermis and a deep skin layer 303 being the subcutis. The epidermis 301 comprises melanin particles that cause the skin 300 to have a certain skin tone as discussed above. Close to the epidermis 301, the dermis 302 comprises the upper or superficial arteriovenous plexus 311, which is a network of fine capillaries supplying the epidermis 301. The reticular plexus 312 is spread more over the central portion of the dermis 302. Finally, the deep plexus 313 is located close to and/or in the subcutis 303. The deep plexus 313 comprises capillaries 314 that supply the hair root 401 with blood. The respective capillaries 314 end in the hair papilla 315. Capillaries of the reticular plexus 312 surround the hair root 401, i.e., extend in the reticular dermis 302.

Fig. 4 is a plot of an intensity spectrum 800 of light emitted by a broad-band flash lamp that may be used in a skin-treatment device. The intensity I_{N} given in arbitrary units (a.u.) is plotted against wavelength λ provided in nanometer (nm). The shown spectrum 800 is understood to have been UV filtered where that a cut-off wavelength of about 550 nm was applied. The spectrum 800 comprises light portions in the visible range up to about 700 nm and in the infrared range above about 700 nm.

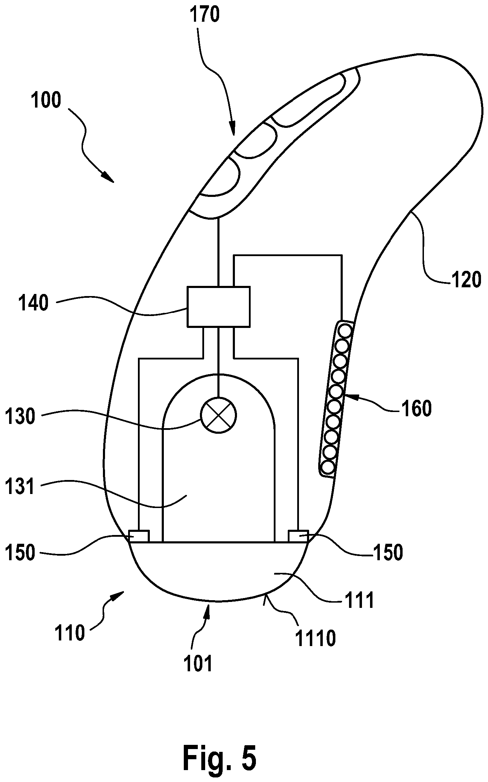

Fig. 5 is a schematic depiction of an example skin treatment device 100 in accordance with the present proposal and disclosure. The skin treatment device 100 comprises a head section 110 and a handle section 120. The head section 110 comprises a light emission window 101 having a window element 111. While other skin treatment devices may comprise a flat window element or the window element may be a frame surrounding an opening of the light emission window, the window element 111 as here shown spans over the complete light emission window 101 and has a convex outer surface 1110. It may be convex in two dimensions, but it may not comprise a curvature in the direction perpendicular to the paper plane. While the concave outer surface 1110 may take any shape, it may be spherical or may be approximated by a portion of a circle, where the radius of the sphere or the circle may be in a range of between 10 mm to 200 mm. If concave in both directions, the curvature may also be different in both directions. The convex outer surface 1110 is intended to be pressed against the treatment surface, typically the human skin during operation. The window element 111 may be made from sapphire glass or any other material suitable to let pass the treatment light, i.e., a material that is translucent or transparent for the treatment light. The convex outer surface 1110 of the window element 111 can be pressed against the skin to squeeze outwards blood from the skin volume to be irradiated. The blood content or blood flow reduction in the skin volume underneath the window element 111 depends on the amount of pressure being applied by the user. A low pressure value will predominantly affect the blood content / blood flow in the papillary loops of the upper plexus. The stronger the pressure becomes, the more blood is pushed away, specifically also in deeper skin areas. With increased pressure, also blood from the reticular plexus is pushed away, which capillaries are arranged around hair roots. As has been already discussed, the optimal pressure value may depend on the skin tone and/or the hair color. Specifically, under some conditions (e.g., blond hair and fair skin) it might be sensible to only push away the blood in the upper plexus so that the light is less absorbed in this upper skin area and more light reaches the hair bulb. The blood in the reticular plexus may then absorb some portion of the light and the respective heat is distributed by the blood flow so that the irradiated area is effectively heated and the heat gradient between hair bulb and surrounding tissue is reduced. This may be helpful as blond hair requires a higher fluence to reach the needed temperature than dark hair as it comprises less melanin. Under other conditions (e.g., dark hair and fair skin) it might be sensible to apply a high force and to push away also the blood of the deeper plexus. Then more light reaches the dark hair bulb and less is absorbed by blood. This may be sensible as dark hair growing in fair skin only requires a relatively low fluence due to the high light absorption by the high melanin content in the hair. In case that the needed fluence can be reduced, the skin treatment device may be able to provide a subsequent light pulse in a shorter period, allowing a user to complete a treatment session in less time.

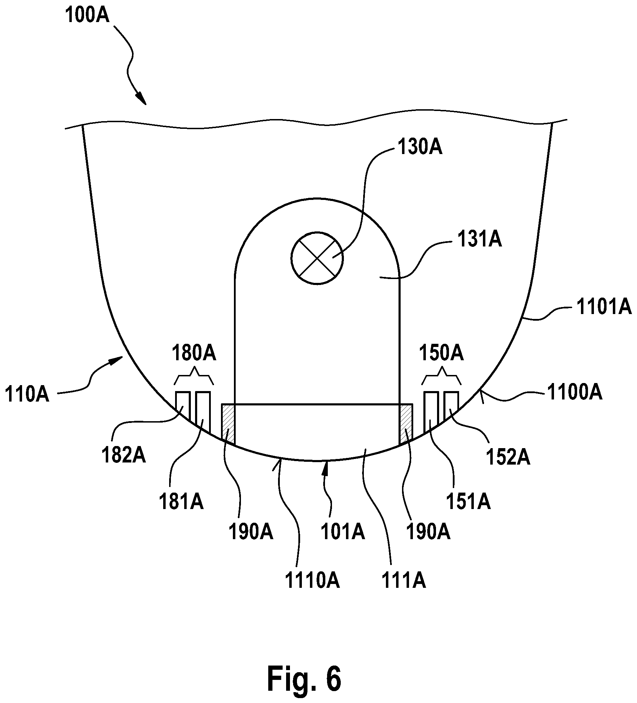

The skin treatment device 100 as shown further comprises a light source 130, a processor 140, at least one first sensor 150, at least one indicator 160 and a user interface 170. The light source 130 is located in a light emission chamber 131 that may have mirror walls so that essentially all light created by the light source 130 is emitted through the light emission window 110 and the window element 111 onto the treatment surface, e.g., human skin. The processor 140 is coupled with the light source 130 to initiate emission of treatment light in case the right conditions are fulfilled. The processor 140 may, as is shown in Fig. 5, be coupled with one or several elements of the skin treatment device 100, e.g., with the at least one first sensor 150 to receive a first sensor signal S₁, with the at least one indicator 160 to trigger indication of a user perceivable signal via the at least one indicator 160, and/or with the user interface 170. The at least one first sensor 150 may be a force or pressure sensor, where in the context of the present application, the term force sensor shall be understood to cover both. The force sensor 150 may be arranged to measure a force value with which the window element 111 is pushed against the skin in operation. The first sensor signal Si may then carry a signal value that can be converted by the processor 140 into a force value using a conversion factor or a calibration table that may be predetermined and may be stored in a memory of the processor 140. The force value may be indicative of a blood / hemoglobin content in the skin area to be treated. Alternatively or additionally, the at least one first sensor 150 may be realized as an optical sensor comprising at least one first sensor light source and at least one light-sensitive detector. The first sensor 150 may then be arranged to provide at least one first sensor signal S₁ being indicative of the blood / hemoglobin content in the skin volume to be treated based on an optical measurement. While a force value may provide an indirect indication of the blood / hemoglobin content, the optical measurement may provide a direct indication of the blood content. This will be discussed in more detail in connection with Fig. 6 further below. The at least one indicator 160 may be realized as at least one visual indicator such as an LED, at least one audible indicator such as a buzzer or a loudspeaker, and/or at least one haptic indicator such as a mechanical vibrator. The user interface 170 may comprise one or a plurality of user operable input elements such as a button, a switch, a touch-sensitive display and/or the like. At least a part of the processor 140, the at least one indicator 160 and/or the user interface 170 may be realized by a separate device, specifically by a mobile device such as a mobile phone or a tablet or the like.

Fig. 6 is a schematic depiction of a front portion of an example skin treatment device 100A, where mainly a head portion 110A is shown. Similar to Fig. 5, the head portion 110A comprises a treatment light source 130A located in a light chamber 131A. A light emission window 101A is covered with a window element 111A that is here smoothly integrated into a housing 1101A, where "smoothly" means that the housing 1101A has an outer surface 1100A that around the window element 111A continues the convex outer surface 1110A of the window element 111A so that there is essentially no step or discontinuity between the convex outer surface 1110A of the window element and the outer surface 1100A of the housing 1101A. This shall not be understood as limiting as the transition between the window element 111A and the outer surface 110A may comprise a step and/or a change in curvature without deviating from the general concept.

In the shown embodiment, cooling elements 190A are coupled with the window element 111A so that the window element 111A is actively cooled. While here two cooling elements 190A are shown, it is sufficient that one cooling element 190A is present. The cooling element/cooling elements 190A may be realized as a Peltier element / Peltier elements, i.e., as a thermoelectric cooler (TEC). Cooling of the window element 111A may reduce the heat sensation felt by users of a skin treatment device, specifically if the skin treatment device is realized as an IPL device.

The skin treatment device 100A comprises a first sensor 150A that comprises a first sensor light source 151A and a first light-sensitive detector 152A for performing a hemoglobin content measurement as discussed before. The skin treatment device 100A may further comprise a second sensor 180 comprising a first light source 181A and a first light-sensitive detector 182A. In some instances, the first sensor 150A and the second sensor 180A together form the first sensor, which simply shall mean that the first sensor may comprise several individual sensors (one of which may be a force sensor). A first sensor comprising two light sources, e.g., one emitting at 580 nm and the other at 680 nm may be used to determine the hemoglobin content in the skin as at these wavelengths the absorption of hemoglobin is about at its maximum and about at its minimum in the sensible wavelength range (see Fig. 1) - this holds only for oxyhemoglobin and not for hemoglobin. At the same time, the absorption by melanin changes not as strongly. This is considered as sensible for determining the blood content. In case of two or more different light sources, one light sensitive sensor may be used that is sensitive for all wavelengths or wavelength ranges emitted by the two or more light sources.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A skin treatment device comprising:
a treatment light source;
a light emission window comprising a window element arranged for getting into skin contact during operation;
a first sensor arranged for providing a first sensor signal indicative of an amount of hemoglobin in a skin area underneath the light emission window; and
a processor coupled with the first sensor for communicating the first sensor signal in a user-perceptible manner by means of an indicator and/or for controlling a function of the skin treatment device in dependence on the first sensor signal.

2. The skin treatment device in accordance with claim 1, the window element having a convex outer surface intended for getting into contact with the skin during operation, the window element being essentially light-transmissive for the treatment light, in particular wherein the window element is a glass element.

3. The skin treatment device in accordance with claim 2, wherein a housing of the skin treatment device around at least a portion of the window element is shaped to continue the convex shape of the window element.

4. The skin treatment device in accordance with one of claims 1 to 3, wherein the first sensor comprises an optical sensor comprising at least a first light source and at least a first light-sensitive detector.

5. The skin treatment device in accordance with claim 4, wherein the first light source is a monochromatic or essentially monochromatic light source such as a laser diode or an LED or the first light source comprises a band-pass filter, preferably wherein the light emitted by the first light source encompasses light having a first wavelength in the range of between 380 nm and 620 nm such as 580 nm or in the range of between 620 nm and 1200 nm such as a 680 nm.

6. The skin treatment device in accordance with claim 4 or claim 5, wherein the first sensor comprises a second light source being a monochromatic or essentially monochromatic light source such as a laser diode or an LED or the second light source comprises a band-pass filter, wherein the light emitted by the second light source encompasses light having a second wavelength in the range of between 380 nm and 620 nm such as 580 nm or in the range of between 620 nm and 1200 nm such as a 680 nm.

7. The skin treatment device in accordance with one of claims 1 to 6, wherein the first sensor comprises a force sensor arranged for measuring a force with which the window element is pushed against the skin.

8. The skin treatment device in accordance with one of claims 1 to 7, further comprising a second sensor for measuring a skin color and/or for measuring or determining a hair color.

9. The skin treatment device in accordance with one of claims 1 to 8, further comprising a user interface allowing a user to input at least one of a skin color or a hair color.

10. The skin treatment device in accordance with one of claims 1 to 9, wherein the processor is arranged to determine an amount of hemoglobin present in the skin underneath the light emission window based on the first sensor signal and to compare the determined amount of hemoglobin present in the skin underneath the light emission window with a threshold amount of hemoglobin, in particular wherein the threshold amount of hemoglobin depends on at least one of the measured or determined and/or inputted skin color or hair color, further preferably wherein the processor is arranged to inhibit an emission of treatment light from the treatment light source when the determined amount of hemoglobin present in the skin underneath the light emission window is above the threshold amount of hemoglobin.

11. The skin treatment device in accordance with one of claims 7 to 10, wherein the processor is arranged to determine a force value with which the window element is pushed against the skin based on the first sensor signal and to compare the determined force value with a threshold force value with which the window element shall be pushed against the skin, preferably wherein the threshold force value depends on at least one of the skin color or the hair color.

12. The skin treatment device in accordance with claim 11, wherein the processor is arranged to indicate in a user-perceptible manner the threshold force value with which the window element shall be pushed against the skin and preferably to indicate in a user-perceptible manner the force value with which the window element is pushed against the skin and/or wherein the processor is arranged to indicate in a user-perceptible manner a sufficiency of the force value with which the window element is pushed against the skin for achieving an intended level of hemoglobin reduction in the skin underneath the light emission window.

13. The skin treatment device in accordance with claim 11 or claim 12, wherein the processor is arranged to inhibit emission of treatment light from the treatment light source if the force value with which the light emission window is pushed against the skin is below the threshold force value.

14. The skin treatment device in accordance with one of claims 1 to 13, wherein the window element is cooled, in particular wherein at least one cooling element is provided in contact with the window element.

15. A method of applying light to skin for achieving a cosmetic effect, the method comprising the steps of:
- pushing a light emission window of a skin treatment device, the light emission window comprising a window element, against the skin;
- measuring a first sensor signal indicative of an amount of hemoglobin present in at least a portion of the skin underneath the light emission window; and
∘ indicating the determined sensor signal in a user-perceptible manner; and/or
∘ controlling a function of the skin treatment device in dependence on the determined sensor signal.
